# EUROPEAN PATENT APPLICATION

(11) **EP 2 647 390 A1**
(43) Date of publication of application: **09.10.2013**
(21) Application number: 12382135.7
(22) Date of filing: 02.04.2012
(51) Int. Cl.: A61K 47/48, A61K 39/135

(54) **Peptide vaccines for the prevention of foot-and-mouth disease**

(71) Applicant: Universitat Pompeu-Fabra, 08002 Barcelona (ES); CONSEJO SUPERIOR DE INVESTIGACIONES CIENTIFICAS, 28006 Madrid (ES); Instituto Nacional de Investigación y Tecnología Agraria y Alimentaria (INIA), 28040 Madrid (ES)
(72) Inventor: Andreu Martínez, David, E-08006 Barcelona (ES); Bárcena Del Riego, Juan, E-28110 Algete-Madrid (ES); Blanco Lavilla, Esther, E-28110 Algete- Madrid (ES); Cubillos Zapata, Carolina, E-28002 Madrid (ES); García De La Torre, Beatriz, E-08004 Barcelona (ES); Monsó Olivares, Marta, E-08003 Barcelona (ES); Sobrino Castelló, Francisco, E-28033 Madrid (ES)
(74) Representative: ABG Patentes, S.L.

(57) **Abstract**

The invention relates to a dendrimeric peptide construct of formula (I) wherein: T is a T epitope of the FMDV, bound to the lysine core by its N-terminal end, B is a B epitope of the FMDV, W is a linker group, Z is Cys bound to the linker group W through its sulphur atom or Lys bound to the linker group W through its ε amino group, X is absent when B is bound to the α-carboxylic group of Z by its N-terminal end or is an acyl group when B is bound to the α-amino group of Z by its C-terminal end. The invention also refers to the use of said constructs as an immunogen for the prevention of foot-and-mouth disease in an animal.

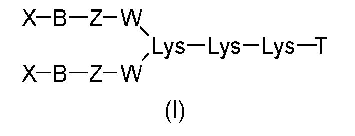

SEQUENCE LISTING

<110>  Universitat Pompeu Fabra
       Consejo Superior de Investigaciones Científicas
       Instituto Nacional de Investigación y Tecnología Agraria y
       Alimentaria

<120>  PEPTIDE VACCINES FOR THE PREVENTION OF FOOT-AND-MOUTH DISEASE

<130>  P8244EP00

<160>  6

<170>  PatentIn version 3.5

<210>  1
<211>  15
<212>  PRT
<213>  Artificial Sequence

<220>
<223>  residues 21-35 of protein 3A of foot-and-mouth disease virus
       serotype CS8

<400>  1

Ala Ala Ile Glu Phe Phe Glu Gly Met Val His Asp Ser Ile Lys
1               5                   10                  15


<210>  2
<211>  19
<212>  PRT
<213>  Artificial Sequence

<220>
<223>  residues 141-159 of protein VP1 of foot-and-mouth disease virus
       serotype O/UK/01

<400>  2

Pro Val Thr Asn Val Arg Gly Asp Leu Gln Val Leu Ala Gln Lys Ala
1               5                   10                  15


Ala Arg Thr



<210>  3
<211>  19
<212>  PRT
<213>  Artificial Sequence

<220>
<223>  residues 141-159 of protein VP1 of foot-and-mouth disease virus
       serotype O1Campos

<400>  3

Ala Val Pro Asn Val Arg Gly Asp Leu Gln Val Leu Ala Gln Lys Val
1               5                   10                  15


Val Arg Thr

<210>  4
<211>  20
<212>  PRT
<213>  Artificial Sequence

<220>
<223>  residues 139-158 of protein VP1 of foot-and-mouth disease virus
       serotype A2001 TrenqueLauquen

<400>  4

Ser Gly Leu Ser Arg Arg Gly Asp Leu Gly Ser Leu Ala Ala Arg Val
1               5                   10                  15

Ala Lys Ala Leu
            20

<210>  5
<211>  20
<212>  PRT
<213>  Artificial Sequence

<220>
<223>  residues 139-158 of protein VP1 of foot-and-mouth disease virus
       serotype A24Cruz

<400>  5

Gly Gly Ser Gly Arg Arg Gly Asp Met Gly Ser Leu Ala Ala Arg Val
1               5                   10                  15

Val Lys Gln Leu
            20

<210>  6
<211>  20
<212>  PRT
<213>  Artificial Sequence

<220>
<223>  residues 141-160 of protein VP1 of foot-and-mouth disease virus
       serotype O1BFS

<400>  6

Val Pro Asn Leu Arg Gly Asp Leu Gln Val Leu Ala Gln Lys Val Ala
1               5                   10                  15

Arg Thr Leu Pro
            20

## Description

### Field of the Invention

The invention relates to dendrimeric peptide constructs based on a lysine core that comprise both B and T epitopes of foot-and-mouth disease virus (FMDV). The invention also refers to a process for preparing these constructs, to compositions comprising the same and to their use in the prevention of foot-and-mouth disease in an animal.

### Background of the Invention

The foot-and-mouth disease virus (FMDV) is a picornavirus that produces a highly transmissible and devastating disease in cloven-hoofed animals. Foot-and-mouth disease is a severe plague for animal farming, since it is highly infectious. Its containment demands considerable efforts in vaccination, strict monitoring, trade restrictions and quarantines, and occasionally the elimination of millions of animals.

The FMDV particle contains a positive-strand RNA molecule of approximately 8500 nucleotides, enclosed within an icosahedral capsid comprising 60 copies of each of the four viral proteins, VP1-4. The genomic RNA encodes a single polyprotein from which the different viral proteins are generated, through a proteolitic process, due to the action of different viral proteases, which include nine mature non-structural proteins (NSPs). Each of these NSPs, as well as some of the precursor polypeptides, are involved in functions relevant to the life cycle multiplication of a virus in infected cells. FMDV shows a high genetic and antigenic variability, which is reflected in the existence of the seven serotypes (O, A, C, SAT1, SAT2, SAT3 or ASIA1) and the numerous variants.

On the other hand, the antigenic structure of the FMDV, as recognized by B lymphocytes, has been characterized in detail. The main epitope B, which is considered to be the virus' main immunogen, is continuous and immunodominant (Site A) and is located in the GH loop, around positions 140 to 160 of protein VP1 (e.g. Bittle et al, Nature 1982, vol. 298, p. 30-33). This epitope has been widely used as an immunogen in various studies (e.g. Brown F., Vaccine 1992, vol. 10(14), p. 1022-1026). In 1986, DiMarchi and colleagues (DiMarchi et al., Science 1986, vol. 232, p. 639-641) reported protection against viral challenge infection in cattle immunised with a peptide in which the VP1 residues 140-160 were colinearly synthesised with the residues corresponding to positions 200-213 of the same protein. However, further studies involving a significant number of animals have pointed out that this peptide epitope provides limited protection in natural FMDV hosts (e.g. Barteling S.J, Adv. Biotechnol. Processes 1988, vol. 10, p. 25-60).

Furthermore, several T-cell epitopes recognised by lymphocytes from FMDV infected pigs have been identified in non-structural proteins (e.g. Blanco E. et al., Journal of Virology 2001, vol. 75, p. 3164-3174). Among these epitopes, epitope T of protein 3A [21-35], synthesised colinearly with epitope B of VP1 [137-156], was able to efficiently stimulate lymphocytes from an infected animal and induced significant levels of serotype-specific anti-FMDV activity in vitro.

On one hand, the problem with the foot-and-mouth disease virus, which has prompted such health concerns, is that it is very contagious. The infected animals release the virus through their saliva (which is produced in abundance as a result of the disease), their urine and faeces: when the blisters burst, the virus is also released from inside. Milk and meat contain the virus, too. This means that anything which is contact with the infected animal will be easily contaminated. Likewise, the virus is very resistant under particular conditions. Therefore, it is necessary to take strict precautionary measures to avoid the contagion and spread of this illness which, although not being dangerous for human health, can cause severe economic losses.

Currently, foot-and-mouth disease is mainly controlled through the use of vaccines based on the inactivated virus. Both FMDV infections and the immunisation based on these vaccines usually elicit high levels of specific circulating antibodies that are correlated with the protection from the related homologous virus and antigenically related virus.

Despite the progress in recent years, the vaccines known and used until now show several disadvantages, such as the requirement of a cold chain to preserve their stability, the need for periodic revaccinations, the risk of infectious virus release during its production, and its non-identifying nature, in other words, the difficulty of serologically distinguishing infected animals from those that have been vaccinated.

Peptide dendrimers containing epitopes of FMDV have been disclosed. Francis et al. (Immunology 1991, 73, 249-254) refer to multiple antigenic peptide (MAP) systems, having dimeric, tetrametic or octameric structure, comprising a B epitope of FMDV (amino acids 141-160 of protein VP1). EP 2 053 056 describes tetrameric peptide constructs based on a lysine core comprising both B and T epitopes of FMDV.

Despite the above prior art, further FMD vaccines are still needed. Specially, systems with improved immunogenicity, easier to synthesized or easier to purify would be desirable.

### Summary of the Invention

Inventors have found that dimeric peptide constructs based on a lysine core and containing a T epitope bound to the C-terminal residue of the lysine core and two B epitopes bound to the N-terminal residues of the lysine core are easier to obtain and purify than the corresponding tetrameric peptide construct. Lysine units in the lysine core are bound to each other through an amide bond between the α-amino group of a Lys and the α-carboxyl group of another Lys.

In addition, the inventors have surprisingly found that these dimeric peptide constructs present improved immunogenic activity compared to the corresponding tetrameric peptide construct as disclosed in EP 2 053 056 (see Figures 1 and 2). This is remarkable, especially in view of Francis et al. (Immunology 1991, 73, 249-254) where tetrameric structures are disclosed as having an optimal immune response, being five- to ten-fold more immunogenic than the corresponding dimer.

Thus, in a first aspect, the invention is directed to a dendrimeric peptide construct of formula (l) 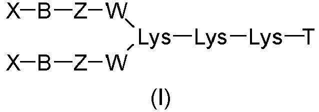 wherein
- T: is a T epitope of the FMDV, bound to the lysine core by its N-terminal end;
- B: is a B epitope of the FMDV;
- W: is a linker group;
- Z: is Cys bound to the linker group W through its sulphur atom or Lys bound to the linker group W through its amino group,
- X: is absent when B is bound to the α-carboxylic group of Z by its N-terminal end or is an acyl group when B is bound to the α-amino group of Z by its C-terminal end.

In a second aspect, the invention is directed to a process for preparing a dendrimeric peptide construct of formula (l).

In a third aspect, the invention refers to a composition comprising a dendrimeric peptide construct of formula (l) and a veterinarilly acceptable adjuvant, carrier or diluent.

Further aspects of the invention are directed to a dendrimeric peptide construct of formula (l) for use in medicine, and to a dendrimeric peptide construct of formula (l) for use in the prevention of foot-and-mouth disease in an animal.

### Brief Description of Figures

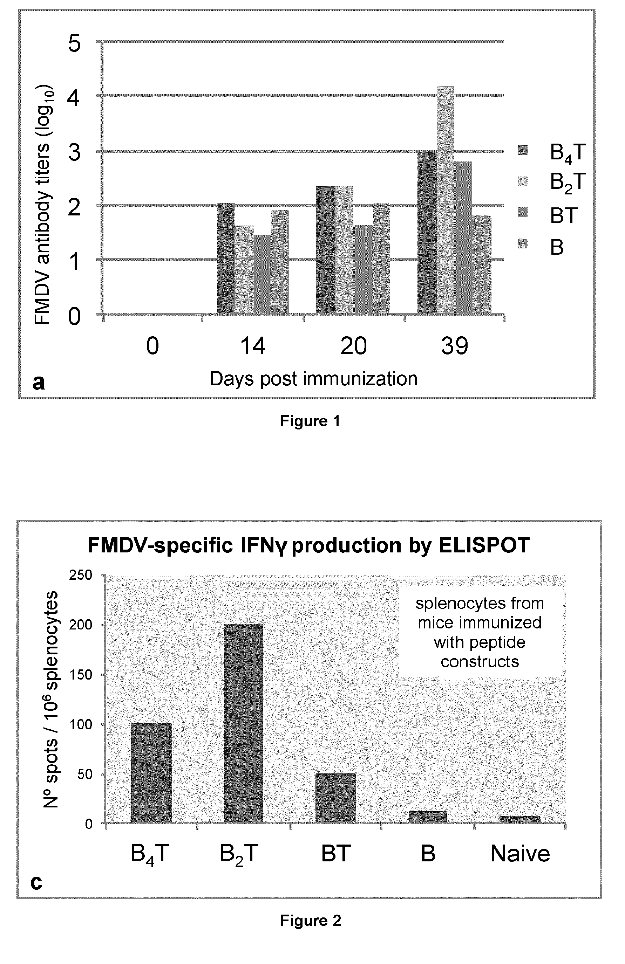

Figure 1 shows the antibody response induced by the dimeric peptide construct of the invention (B₂T) compared to the corresponding tetrameric peptide construct (B₄T), linear peptide (BT) and epitope B alone. B₂T refers to a construct of formula (I) according to the present invention, wherein X is Ac, B is SEQ ID NO: 2, Z is Cys, W is - CH₂-CO- and T is SEQ ID NO: 1. B₄T refers to a tetrameric peptide construct as described in EP 2053056 wherein X, B, Z, W and T are as defined for B₂T. BT refers to a linear peptide formed by SEQ ID NO: 2 bonded to SEQ ID NO: 1. B refers to SEQ ID NO: 2.
Figure 2 represents the IFN-γ expression induced by the dimeric peptide construct of the invention (B₂T) compared to the corresponding tetrameric peptide construct (B₄T), linear peptide (BT) and epitope B alone. B₂T, B₄T, BT and B are as defined for Figure 1.

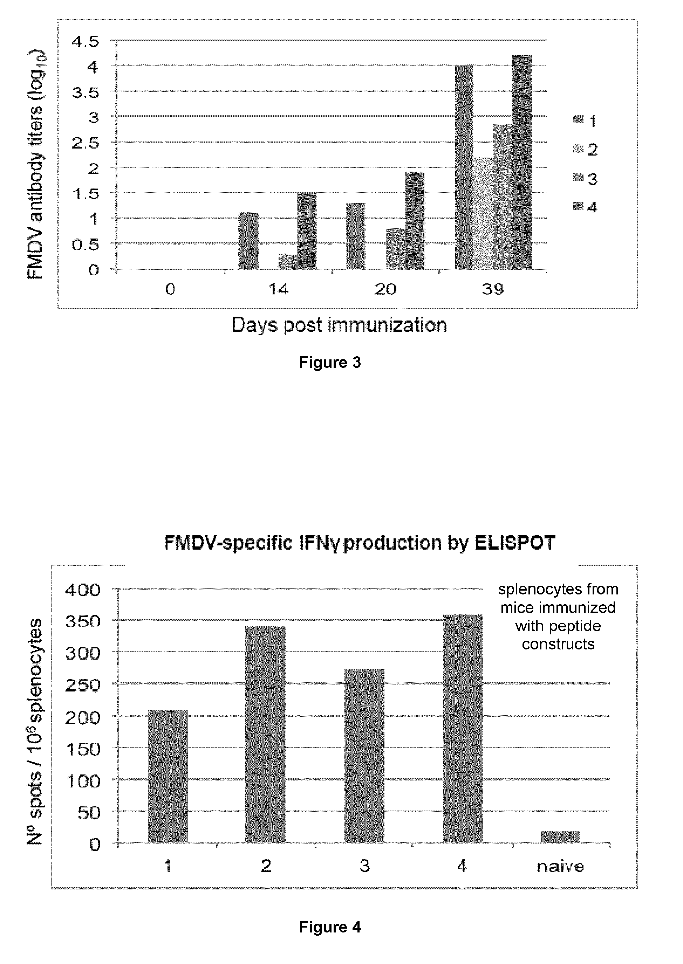

Figure 3 shows the antibody response induced by constructs **1-4** of the invention.
Figure 4 shows represents the IFN-γ expression induced by constructs **1-4** of the invention.

### Detailed Description of the Invention

In a first aspect, the invention is directed to a dendrimeric peptide construct of formula (l) 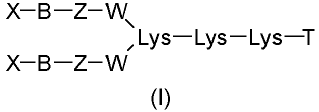 wherein
- T: is a T epitope of the FMDV, bound to the lysine core by its N-terminal end;
- B: is a B epitope of the FMDV;
- W: is a linker group;
- Z: is Cys bound to the linker group W through its sulphur atom or Lys bound to the linker group W through its amino group,
- X: is absent when B is bound to the α-carboxylic group of Z by its N-terminal end or is an acyl group when B is bound to the α-amino group of Z by its C-terminal end.

The core of lysines represented by 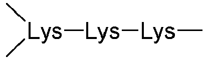 corresponds to the following chemical structure: 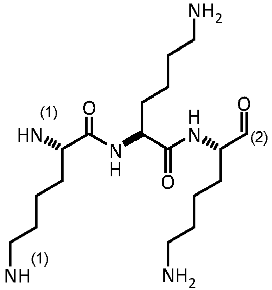 wherein the amino groups marked with (1) are linked to the linker group W and the carbonyl group marked with (2) forms part of the peptidic bond linking with epitope T.

T and B epitopes of the FMDV are well known in the art (e.g. Veterinary Research 2001, 32:1-30), or they can be identified empirically (e.g. using PEPSCAN or similar methods, Geysen et al. (1984) PNAS USA 81:3998, Carter (1994) Methods Mol Biol 36:207), or can be predicted (International Journal of Biotechnology Applications 2009, 1:1-3; Journal of General Virology 2011, 2297; BMC Bioinformatics 2009, 10:302).

In a particular embodiment, T epitope of the FMDV is a T epitope of the non-structural protein 3A, 3B or 3C of the FMDV.

In a further embodiment, T epitope of the FMDV comprises residues 21-35 of protein 3A of FMDV serotype C (CS8 strain), or the corresponding amino acid residues of another strain of serotype C or of another serotype of FMDV, such as serotypes A, C, SAT1, SAT2, SAT3 or ASIA1.

In a preferred embodiment, T epitope comprises the amino acid sequence AAIEFFEGMVHDSIK (SEQ ID NO: 1).

Further T epitopes may be identified using *in vitro* T-cell stimulation techniques of component proteins, protein fragments and peptides to identify appropriate sequences (Ann. Rev. Immunol., 1, 465, (1983); "The Year in Immunology, vol. 2", 1986, p. 151; Ann. Rev. Immunol., 5, 477, (1987)).

Preferably, the T epitope is capped at the C-terminus as a carboxamide.

In a particular embodiment, B epitope of the FMDV is a B epitope of the capsid protein VP1, VP2 or VP3 of the FMDV.

The main B epitope of FMDV has been identified as a region called GH loop or antigenic site, which is located approximately between residues 140-160 of VP1. In a further embodiment, B epitope of the FMDV comprises antigenic site A of protein VP1.

In a further embodiment, B epitope of the FMDV comprises residues 141-159 of protein VP1 of FMDV serotype O (O/UK/01, O1Campos, O1BFS strains), or residues 139-158 of protein VP1 of FMDV serotype A (A2001 TrenqueLauquen, A24Cruz strains), or residues 136-153 of protein VP1 of FMDV serotype C (CS8 strain) or the corresponding amino acid residues of another strain of the same serotype or of another serotype of FMDV, such as serotypes SAT1, SAT2, SAT3 or ASIA1.

In a preferred embodiment, B epitope comprises an amino acid sequence selected from the group consisting of PVTNVRGDLQVLAQKAART (SEQ ID NO: 2), AVPNVRGDLQVLAQKVVRT (SEQ ID NO: 3), SGLSRRGDLGSLAARVAKAL (SEQ ID NO: 4), GGSGRRGDMGSLAARVVKQL (SEQ ID NO: 5) and VPNLRGDLQVLAQKVARTLP (SEQ ID NO: 6). More preferably, B epitope comprises an amino acid sequence selected from the group consisting of PVTNVRGDLQVLAQKAART (SEQ ID NO: 2).

T and B epitopes of the FMDV include peptides that, compared to the sequences disclosed herein, include one or more (e.g. 1, 2, 3, 4, 5, 6, 7, 8, 9, 10, etc.) conservative amino acid substitutions i.e. replacements of the amino acid with another which has a related side chain. Such substitutions and modifications are generally well-known to those skilled in the art of peptide chemistry. Genetically-encoded amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine). In general, substitution of single amino acids within these families does not have a major effect on the biological activity. Moreover, the peptides may have one or more (e.g. 1, 2, 3, 4, 5, 6, 7, 8, 9, 10, etc.) single amino acid deletions relative to the sequences disclosed herein. Furthermore, the peptides may include one or more (e.g. 1, 2, 3, 4, 5, 6, 7, 8, 9, 10, etc.) insertions relative to the sequences disclosed herein.

In the present invention, the term "linker group" refers to a bi-functional unit that makes possible to link covalently the sulphydryl or the ε-amino groups of Z with the amino groups of the lysine core.

In a particular embodiment, W is an organic group having from 1 to 20, preferably from 1 to 10, carbon atoms and optionally containing at least one, preferably 1, 2, 3 or 4, heteroatoms selected from N, O and S. Preferably, W is a cyclic or acyclic hydrocarbon group having from 1 to 20, preferably from 1 to 10, carbon atoms wherein optionally one or more, preferably 1, 2, 3 or 4, -CH₂- or -CH- groups are replaced by - CO-, -S-, -O-, -N H- or 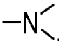 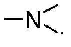 More preferably, W is selected from a group of formula:

-(CH₂)ₙ-CO-,

-CO-(CH₂)ₙ-S-(CH₂)ₘ-CO-, or

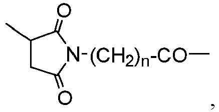

wherein n and m are independently selected from 1, 2, 3, 4, 5 and 6.

In a further embodiment, W is selected from a group of formula:

-CH₂-CO-,

-CO-CH₂-S-(CH₂)₂-CO-, or

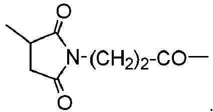

In a particular embodiment, Z is Cys bound to the linker group through the -SH group. In another embodiment, Lys is bound to the linker group through the ε amino group.

In a particular embodiment, B is bound to the α-amino group of Z by its C-terminal end and X is an acyl group, i.e. a group of formula R-CO- wherein R is an alkyl group having 1 to 6, preferably 1 to 3, carbon atoms. More preferably, X is an acetyl group (abbreviated as Ac).

In an embodiment, peptide construct of formula (l) is a construct of formula (la), (lb), (lc) or (ld): 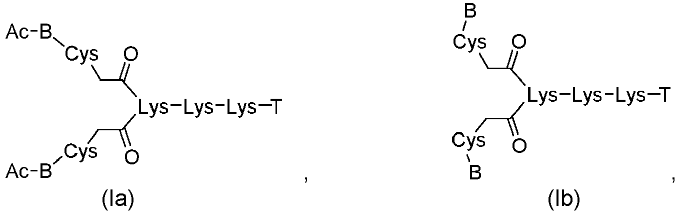 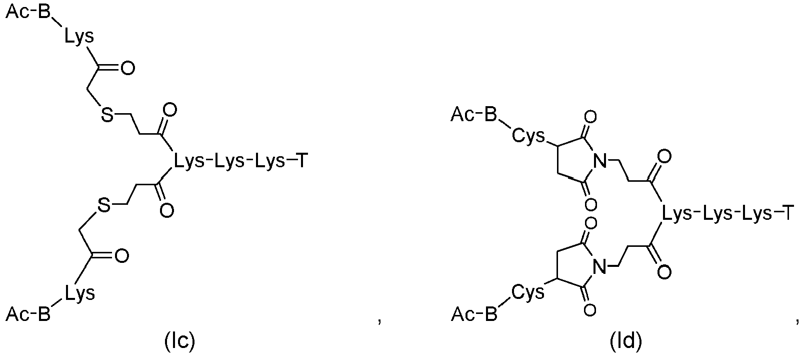 wherein B and T are as defined herein. Formula (lb) refers to a construct wherein B is bound to the carboxylic group of Z (Cys) by its N-terminal end and formulae (la), (lc) and (ld) refer to constructs wherein B is bound to the amino group of Z by its C-terminal end and the N-terminal is acetylated.

In a particular embodiment, peptide construct of formula (l) is a construct of formula (la), (lb), (lc) or (ld) wherein T is a T epitope of the FMDV comprising residues 21-35 of protein 3A of FMDV serotype C (CS8 strain), or the corresponding amino acid residues of another strain of serotype C or of another serotype of FMDV, such as serotypes A, C, SAT1, SAT2, SAT3 or ASIA1. More preferably, it is a construct of formula (la), (lb), (lc) or (ld) wherein T is a T epitope comprising the amino acid sequence AAIEFFEGMVHDSIK (SEQ ID NO: 1).

In a particular embodiment, peptide construct of formula (l) is a construct of formula (la), (lb), (lc) or (ld) wherein B is a B epitope of the FMDV comprising antigenic site A of protein VP1, preferably, residues 141-159 of protein VP1 of FMDV serotype O (O/UK/01, O1Campos, 01BFS strains), or residues 139-158 of protein VP1 of FMDV serotype A (A2001 TrenqueLauquen, A24Cruz strains), or residues 136-153 of protein VP1 of FMDV serotype C (CS8 strain) or the corresponding amino acid residues of another strain of the same serotype or of another serotype of FMDV, such as serotypes SAT1, SAT2, SAT3 or ASIA1. More preferably, it is a construct of formula (la), (lb), (lc) or (ld) wherein B is a B epitope comprising an amino acid sequence selected from the group consisting of PVTNVRGDLQVLAQKAART (SEQ ID NO: 2), AVPNVRGDLQVLAQKVVRT (SEQ ID NO: 3), SGLSRRGDLGSLAARVAKAL (SEQ ID NO: 4), GGSGRRGDMGSLAARVVKQL (SEQ ID NO: 5), VPNLRGDLQVLAQKVARTLP (SEQ ID NO: 6).

In a further embodiment, peptide construct of formula (l) is a construct of formula (la), (lb), (lc) or (ld) wherein T is a T epitope comprising the amino acid sequence AAIEFFEGMVHDSIK (SEQ ID NO: 1) and B is a B epitope comprising an amino acid sequence selected from the group consisting of PVTNVRGDLQVLAQKAART (SEQ ID NO: 2), AVPNVRGDLQVLAQKVVRT (SEQ ID NO: 3), SGLSRRGDLGSLAARVAKAL (SEQ ID NO: 4), GGSGRRGDMGSLAARVVKQL (SEQ ID NO: 5), VPNLRGDLQVLAQKVARTLP (SEQ ID NO: 6), preferably, an amino acid sequence selected from the group consisting of PVTNVRGDLQVLAQKAART (SEQ ID NO: 2).

In a second aspect, the invention refers to a process for preparing a dendrimeric peptide construct of formula (la) or (lb) which comprises the reaction of a compound of formula (II) 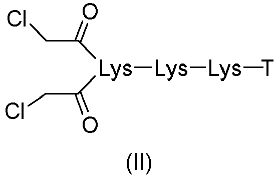 with a compound of formula (III) 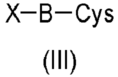 wherein T, B and X are as defined previously,
at a pH between 6 and 7.5.

Alternatively, constructs of formula (lc) can be obtained by a process which comprises the reaction of a compound of formula (IV) 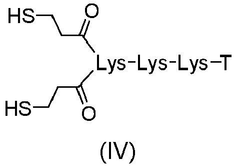 with a compound of formula (V) 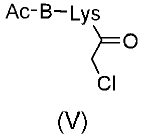 wherein T and B are as defined previously,
at a pH between 6 and 7.5.

Alternatively, constructs of formula (ld) can be obtained by a process which comprises the reaction of a compound of formula (VI) 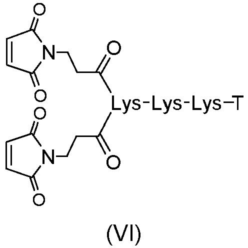 with a compound of formula (III), wherein T and B are as defined previously, at a pH between 6 and 7.

In another embodiment, the compound of formula (III) is in a molar excess of from 2 to 25, preferably from 5 to 20, in respect of the compound of formula (II) or of formula (VI). In an embodiment, the compound of formula (V) is in a molar excess of from 2 to 25, preferably from 5 to 20, in respect of the compound of formula (IV).

Compounds of formula (II), (III), (IV), (V) and (VI) can be obtained by solid phase peptide synthesis.

In a particular embodiment, the compound of formula (II) can be obtained by first synthesizing the T epitope on a solid support, preferably a polymeric resin, using Fmoc chemistry in an automated synthesizer, then the lysine core is introduced by coupling with Fmoc-Lys(Fmoc)-OH and then the α and ε amino groups are deprotected and functionalized with a -W-Cl group. The resulting peptide is deprotected and cleaved from the solid support. Preferably, when W is a -CH₂-CO- group, the deprotected and cleaved peptide is chloroacetylated at α and ε amino groups, preferably by treatment with chloroacetic acid.

In a particular embodiment, compound of formula (IV) can be obtained in a similar way as described for compound (II) where, after coupling with Fmoc-Lys(Fmoc)-OH and deprotection, the resulting peptide is functionalized at α and ε amino groups with a -CO-(CH₂)₂-SH group, preferably by treatment with S-trityl-3-mercaptopropionic acid, and cleaved from the solid support.

In a particular embodiment, compound of formula (VI) can be obtained in a similar way as described for compound (II) where, after coupling with Fmoc-Lys(Fmoc)-OH and deprotection, the resulting peptide is functionalized at α and ε amino groups by using N-maleoyl-β-alanine, and cleaved from the solid support.

In a particular embodiment, compound of formula (III) can be obtained by peptide synthesis on a solid support, preferably a polymeric resin, using Fmoc chemistry in an automated synthesizer. The resulting peptide can be acylated or not at the N-terminal end, then deprotected and cleaved from the solid support.

In a particular embodiment, compound of formula (V) can be obtained in a similar way as described for compound (III) but wherein the C-terminal Lys is deprotected at its ε amino group, then chloroacetylated, preferably by treatment with chloroacetic acid. The resulting peptide is deprotected and cleaved from the solid support.

In another aspect, the invention refers to a veterinary composition that comprises at least a dendrimeric peptide construct of formula (I) as defined herein and a veterinarilly acceptable adjuvant, carrier or diluent. Conventional formulations, adjuvants, carriers and diluents may be employed. Suitable carriers and diluents are known in the art (e.g. McKercher PD et al., "A review of the current status of oil adjuvants in foot-and-mouth disease", Dev. Biol. Stand. 1976, vol. 35, p. 107-112; Dong XN et al., "Candidate multi-peptide-vaccine against classical swine fever virus induced potent immunity with serological marker", Vaccine 2005, vol. 23, p. 3630-3633) and include, for example, Freund's complete and incomplete adjuvant, aluminium hydroxide, saponin, dextrans, mineral oils, neutral oils, vegetable oils, liposomes, polyols, etc.

These compositions may be suitable as immunogenic compositions or as vaccines. The compositions can be administered by any convenient route including any oral or parenteral route, such as subcutaneous, intravenous or intramuscular.

Constructs of formula (l) have been found to present immunogenic properties. Therefore, in another aspect, the invention refers to a dendrimeric peptide construct of formula (l) for use in medicine.

In another aspect, the invention refers to a dendrimeric peptide construct of formula (l) for use in the prevention of foot-and-mouth disease in an animal.

The invention also describes the use of a dendrimeric peptide construct of formula (l) in the preparation of a medicament for the prevention of foot-and-mouth disease in an animal.

The invention also discloses a method of preventing foot-and-mouth disease in an animal comprising administering an effective amount of a dendrimeric peptide construct of formula (l).

Animals to be treated include both domestic and wild animals, such as cattle, pigs, sheep, goats, buffalo, antelope, deer, wild boar, bison or any other cloven-hoofed animal.

The following examples illustrate the invention and should not be considered as limitative of the invention.

### EXAMPLES

### General peptide synthesis protocols.

The linear peptides were synthesized by automated synthesis Fmoc (FastMoc) protocols performed in an AB1433 peptide synthesizer (Applied Biosystems, Foster City, CA) at 0.1 mmol scale on Fmoc-Rink-amide ChemMatrix resin. The side chain functionalities were protected with ^{t}Bu (Asp, Glu, Ser, Thr, Tyr), Boc (Lys, Trp), Pbf (Arg) and Trt (Asn, Gln, His) groups. Eight-fold excess of Fmoc-L-amino acids and HBTU, in the presence of double molar amount of DIEA, were used for the coupling steps, with DMF as solvent. Branching was introduced by the incorporation of Fmoc-Lys(Fmoc)-OH at the appropriate points. After derivatization, peptides were fully deprotected and cleaved with TFA/H₂O/TIS (95:2.5:2.5 v/v, 90 min, RT), and precipitated by addition of chilled diethyl ether, taken up in aqueous AcOH (10% v/v), and lyophilized. Then, they were purified and characterised by analytical HPLC and MALDI-TOF MS as detailed below.

### Functionalization and conjugation of the peptides.

**Conjugates 1 and 2.** Conjugates **1** and **2** correspond to constructs of formula (la) and (lb), respectively, wherein T is SEQ ID NO: 1 and B is SEQ ID NO: 2. These molecules were synthesized using standard thioether ligation.

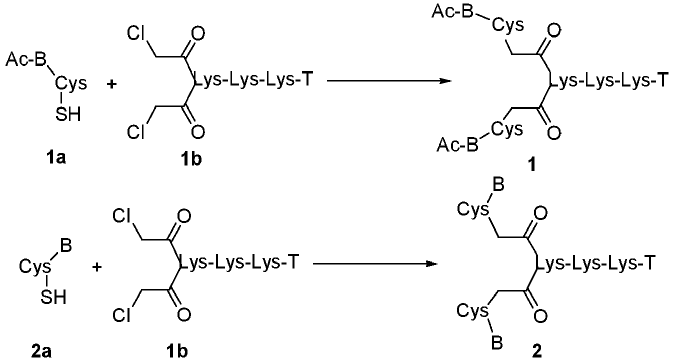

Moiety **1a** has a Cys residue at the C end of the peptide and N-terminal is acetylated. In the case of **2a**, the Cys residue is placed in the N-terminal part of the peptide. The branched compound **1b** was obtained by chloracetylation at the α and ε amino groups of the Lys placed in the N-terminal part of the peptide, using 10 fold excess of chloroacetic acid and DIPCI (1:1). All compounds **(1a, 1b** and **2a)** were cleaved and purified by HPLC before subsequent conjugation. Conjugates **1** and **2** were obtained by dissolving 3.5 mg of the chloroacetylated core **(1b)** in 10 mL of a 0.02 M solution of NaHCO₃, pH 7.5, at 50 °C. Then, 8-fold molar excess of the compound **1a** or **1b** was added portion wise to the previous solution and the pH was adjusting to 7.5. The reaction was monitored by HPLC and MALDI-TOF, analyzing aliquots of the mixtures every hour. When no changes were observed in the HPLC profile, the reaction product was separated by preparative RP-HPLC and then characterized by analytical HPLC and MALDI-TOF MS (Conjugate **1:** Average mass for the [MH]⁺ ion (Da), Calculated: 6521.5; Found: 6523.5; Conjugate **2:** Average mass for the [MH]⁺ ion (Da), Calculated for [MH]⁺ ion: 6521.5; Found: 6519.1). **Conjugate 3.** Conjugate **3** corresponds to constructs of formula (lc), wherein T is SEQ ID NO: 1 and B is SEQ ID NO: 2. This molecule was obtained by reverse thioether approach.

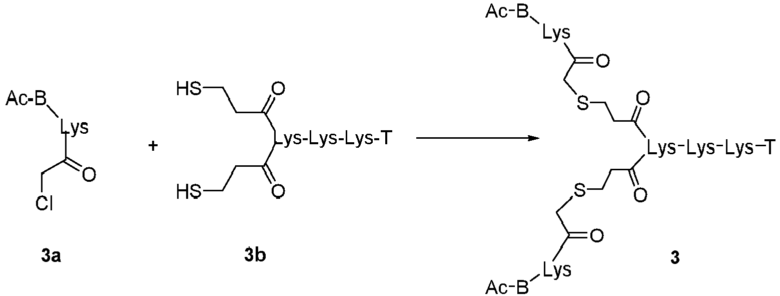

In this case, the B epitope was synthesized with a Fmoc-Lys(Mmt) residue in the C terminal part of the sequence, then it was acetylated, the protecting group Mmt was selectively removed using 1%TFA in DCM and finally the C terminal Lys residue was chloracetylated as described for peptide **1b,** to yield compound **3a.** Compound **3b** was obtained by thio-functionalization with Trt-MPA in DIPCI (1:1). Compounds **3a** and **3b** were removed from the resin and purified, as previously described, and conjugated. The conjugation was performed in the same solution as in standard thioether approach (0.02 M solution of NaHCO₃, pH 7.5). In this approach, 3.5 mg of **3b** were dissolved in 10 mL of said solution and 8-fold molar excess of the **3a** were then added portionwise and the solution stirred at 50 °C. The reaction was monitored and stopped as described above. The mixture was purified by RP-HPLC and dendrimer **3** was characterized by HPLC and MALDI-TOF, as detailed below (Conjugate **3:** Average mass for the [MH]⁺ ion (Da), Calculated: 6749.81; Found: 6746.4).

**Conjugate 4.** Conjugate **4** corresponds to constructs of formula (ld), wherein T is SEQ ID NO: 1 and B is SEQ ID NO: 2.

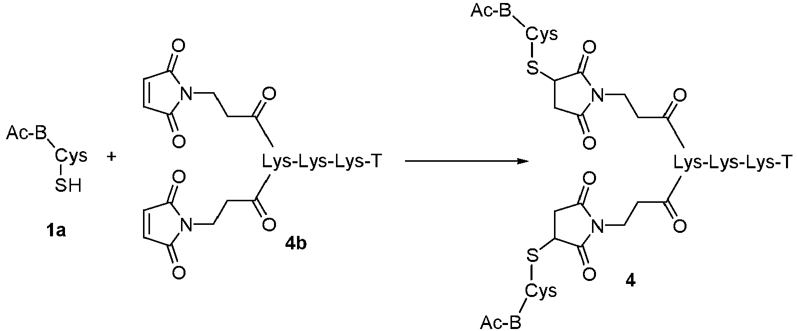

Conjugation was done *via* a maleimide group. The maleimide-functionalization to obtain **4b,** was carried out incorporating N-maleoyl-β-Ala in DIPCI (1:1) at N α and ε of the N terminal Lys residue. Deprotection, cleavage and purification were performed in the same way as before. For the conjugation of both peptide fragments, 4.5 mg of compound **4b,** were dissolved in 1 mL of 50 mM phosphate buffer pH 6 and added to a solution in the same buffer containing stoichiometric amount (double molar) of **1a.** After mixing the solutions, analysis of a small aliquot revealed the total completion of the reaction. Then, the reaction product was purified by RP-HPLC and conjugate **4** was characterized by HPLC and MALDI-TOF (Conjugate **4:** Average mass for the [MH]⁺ ion (Da), Calculated: 6739.5; Found: 6737.1).

### Analysis and purification.

Analytical reversed-phase HPLC was performed on C18 or C8 columns (4.6 x 50 mm, 3 *µ*m, Phenomenex, Torrance, CA) in a model LC-2010A system (Shimadzu, Kyoto, Japan). Solvent A was 0.045% (v/v) TFA in water, solvent B was 0.036% (v/v) TFA in acetonitrile. Elution was done with linear gradients of solvent B into A over 15 min at 1 mL/min flow rate, with UV detection at 220 nm. Preparative HPLC was performed on C18 (21.2 x 250 mm, 10 *µ*m, Phenomenex) or on C8 column (10 x 250 mm, 10 µm, Phenomenex) in a Shimadzu LC-8A instrument. Solvents A and B were 0.1% TFA (v/v) in water and acetonitrile, respectively, and elution was again with linear gradients of solvent B into A over 35 min, at 25 or 5 mL/min flow rate for C18 or C8 separations, respectively, with UV detection at 220 nm. Preparative fractions of satisfactory purity (>95%) by analytical HPLC were pooled and lyophilized. The purified peptides and MAPs were characterized by MALDI-TOF MS in a Voyager DE-STR instrument (Applied Biosystems), R-hydroxycinnamic acid matrixes. MS spectra were performed in the reflector mode for the linear peptide epitopes and the divalent Lys core; for larger-sized analytes, the linear mode was used.

### Immunological assays.

Mice immunization and Ab production. Groups of 5 Swiss mice were subcutaneously immunized with 100 µg of each conjugate in Freund's adjuvant. One identical boost emulsified in incomplete Freund's adjuvant was administered after 20 days. The animals were sacrificed after 39 days of the first dose. Blood samples were taken at 0, 15, 20 and 39 days to study antibody responses to FMDV in sera by ELISA. ELISA plates were coated with purified virus (OUK2001), incubated at 4 °C overnight with 1/320 in PBS (50 µl/well). Free active sites were blocked using blocking buffer (PBS buffer with 0.05% Tween and 5% milk) incubating at 37 °C for 1 h. Sera diluted in blocking buffer were preincubated at room temperature for 15 min and added to the plate (50 µl/well) leaving it for 1 h at 37 °C. After further washing, 50 µL/well of zymed anti-mouse antibodies in blocking buffer was added and plates incubated for 45 min more The amount of coupled conjugate was determined by incubation with 100 µl/well of soluble 3,3',5,5'-tetramethylbenzidine for 15 min. at room temperature. Finally, the reaction was stopped with 100 µL/well of 1M sulphuric acid and the absorbance was determined at 450 nm. Antibody titres are expressed as the log10 of serum dilutions giving an optical density value (OD) equal to the mean plus 2 standard deviations recorded for the negative control (serum collected at day 0). In all cases the standard deviations were < 1.0.

### IFN-γ expression.

Freshly splenocytes were stimulated in vitro with autologous synthetic peptides or FMDV. After 24 hours of incubation, cells were harvested and their activation was analyzed by IFN-y ELISPOT. For this purpose the splenocytes were isolated and transferred to Immobilon-P hydrophobic PVDF plates from Millipore (S2EM004M99) which had been coated with 100 µl of 5 µg/ml anti-mouse Mab BD-51-2525KZ. The cells were incubated for 48 hrs at 37°C and the assay continued using reagents supply by BD Biosciences and following their technical recommendations. Spots appeared at the sites of cytokine localization, with each individual spot representing an individual IFN-y secreting cell. The number of spots was quantified.

## Claims

1. A dendrimeric peptide construct of formula (l) 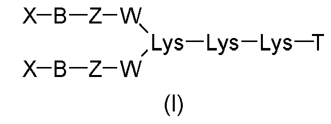 wherein
T is a T epitope of the FMDV, bound to the lysine core by its N-terminal end;
B is a B epitope of the FMDV;
W is a linker group;
Z is Cys bound to the linker group W through its sulphur atom or Lys bound to the linker group W through its ε amino group,
X is absent when B is bound to the α-carboxylix group of Z by its N-terminal end or is an acyl group when B is bound to the α-amino group of Z by its C-terminal end.

2. The dendrimeric construct according to claim 1, wherein W is selected from a group of formula:
-(CH₂)ₙ-CO-,
-CO-(CH₂)ₙ-S-(CH₂)ₘ-CO-, or 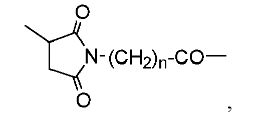
wherein n and m are independently selected from 1, 2, 3, 4, 5 and 6.

3. The dendrimeric construct according to any previous claim, wherein T is a T epitope of the non-structural protein 3A, 3B or 3C of the FMDV.

4. The dendrimeric construct according to claim 3, wherein T is a T epitope of the FMDV comprising residues 21-35 of protein 3A of FMDV serotype C, or the corresponding amino acid residues of another serotype of FMDV.

5. The dendrimeric construct according to claim 4, wherein T comprises the amino acid sequence AAIEFFEGMVHDSIK (SEQ ID NO: 1).

6. The dendrimeric construct according to any previous claim, wherein B is a B epitope of the capsid protein VP1, VP2 or VP3 of the FMDV.

7. The dendrimeric construct according claim 6, wherein B is a B epitope of the FMDV comprising antigenic site A of protein VP1.

8. The dendrimeric construct according claim 7, wherein B is a B epitope of the FMDV comprising residues 141-159 of protein VP1 of FMDV serotype O, or residues 139-158 of protein VP1 of FMDV serotype A, or residues 136-153 of protein VP1 of FMDV serotype C or the corresponding amino acid residues of another serotype of FMDV.

9. The dendrimeric construct according claim 8, wherein B comprises an amino acid sequence selected from the group consisting of PVTNVRGDLQVLAQKAART (SEQ ID NO: 2), AVPNVRGDLQVLAQKVVRT (SEQ ID NO: 3), SGLSRRGDLGSLAARVAKAL (SEQ ID NO: 4), GGSGRRGDMGSLAARVVKQL (SEQ ID NO: 5) and VPNLRGDLQVLAQKVARTLP (SEQ ID NO: 6).

10. The dendrimeric construct according to any previous claim which is selected from a construct of formula (la), (lb), (lc) or (ld): 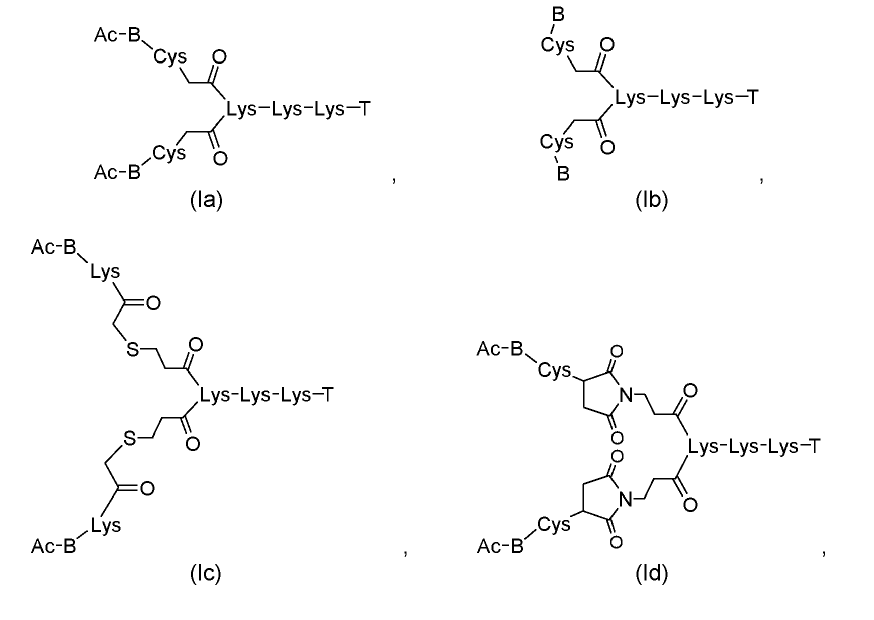 wherein B and T are as defined in any of claims 1-9.

11. The dendrimeric construct according to claim 10 which is selected from a construct of formula (la), (lb), (lc) or (ld) wherein T is SEQ ID NO: 1 and B is SEQ ID NO: 2.

12. A process for preparing a dendrimeric peptide construct of formula (la) or (lb) as defined in any of claim 10, which comprises the reaction of a compound of formula (II) 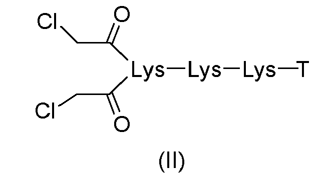 with a compound of formula (III) 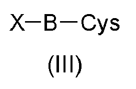 wherein T, B and X are as defined in claim 10,
at a pH between 6 and 7.5.

13. A veterinary composition that comprises a dendrimeric peptide construct of formula (l) as defined in any of claims 1 to 11 and a veterinarilly acceptable adjuvant, carrier or diluent.

14. A dendrimeric peptide construct of formula (l) as defined in any of claims 1 to 11 for use in medicine.

15. A dendrimeric peptide construct of formula (l) as defined in any of claims 1 to 11 for use in the prevention of foot-and-mouth disease in an animal.
